(19) Europäisches Patentamt
European Patent Office
Office européen des brevets

(11) Numéro de publication : **0 348 280 B1**

# (12) FASCICULE DE BREVET EUROPEEN

(45) Date de publication du fascicule du brevet : **14.04.93 Bulletin 93/15**

(51) Int. Cl.$^5$ : **A61K 7/13**

(21) Numéro de dépôt : **89401696.3**

(22) Date de dépôt : **16.06.89**

## (54) Procédés de teinture de fibres kératiniques à base de 5,6-dihydroxy-indole ou d'un dérivé d'indole et d'au moins un sel de terre rare et compositions de mise en oeuvre.

(30) Priorité : **21.06.88 LU 87256**

(43) Date de publication de la demande : **27.12.89 Bulletin 89/52**

(45) Mention de la délivrance du brevet : **14.04.93 Bulletin 93/15**

(84) Etats contractants désignés : **AT BE CH DE FR GB GR IT LI NL SE**

(56) Documents cités :
**EP-A- 0 271 186**
**WO-A-88/01162**
**DE-A- 3 701 044**
**FR-A- 2 327 761**
**GB-A- 2 132 642**
**GB-A- 2 207 443**

(73) Titulaire : **L'OREAL**
**14, Rue Royale**
**F-75008 Paris (FR)**

(72) Inventeur : **Garoche, Didier**
**28bis rue Gabriel Péri**
**F-92300 Levallois-Perret (FR)**
Inventeur : **Grollier, Jean-François**
**16bis Boulevard Morland**
**F-75004 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 348 280 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux procédés de teinture des fibres kératiniques et en particulier des cheveux humains, à base de 5,6-dihydroxyindole et/ou d'un dérivé d'indole et d'au moins un sel de terre rare et aux compositions utilisées dans ces procédés.

La biosynthèse naturelle des eumélanines ou pigments insolubles noirs s'effectue en plusieurs étapes par polymérisation des produits d'oxydation d'un acide aminé : la tyrosine. L'un de ces produits d'oxydation est le 5,6-dihydroxyindole qui s'oxyde à son tour en eumélanine.

EP 271 186 décrit l'utilisation, pour la teinture des cheveux ou de la peau, d'oxydants métalliques, plus particulièrement sous forme de périodates.

Il est bien connu de teindre les fibres kératiniques humaines avec du 5,6-dihydroxyindole, notamment par des procédés qui utilisent les sels métalliques des groupes III à VIII du tableau périodique et en particulier un sel cuivrique. Ces derniers accélèrent le processus de polymérisation oxydante du dérivé d'indole.

Les sels métalliques sont généralement appliqués dans une étape séparée de celle de l'application de la composition à base de 5,6-dihydroxyindole.

Ces procédés de l'état antérieur de la technique proposent d'obtenir des teintures de couleur noire intense dans des temps courts à l'aide de sels cuivriques, la gamme des nuances claires ou intermédiaires précédant le noir ne pouvant être obtenue que dans une étape ultérieure, par éclaircissement du noir au moyen d'une solution de peroxyde d'hydrogène.

Ces mêmes procédés de l'état antérieur de la technique font apparaître que les promoteurs métalliques mentionnés ci-dessus permettent d'obtenir des teintures dans des nuances de gris plus ou moins foncées, sans pouvoir augmenter la profondeur de la couleur obtenue par une exposition plus longue des cheveux à la teinture ou des concentrations plus importantes du promoteur métallique.

La demanderesse vient de découvrir que contrairement à ce qui était possible avec les sels métalliques utilisés dans l'art antérieur et plus particulièrement un sel cuivrique, on pouvait teindre progressivement les cheveux directement dans des nuances claires ou plus ou moins foncées et avec la possibilité d'obtenir des noirs intenses si on le désire, les nuances claires et intermédiaires étant obtenues sans avoir recours à une étape ultérieure supplémentaire d'éclaircissement par le peroxyde d'hydrogène.

Une telle teinture est possible grâce à l'utilisation de sels de terres rares mis en oeuvre, soit dans le cadre d'un prétraitement ou d'un post-traitement, avant ou après l'application de la composition à base de 5,6-dihydroxyindole et/ou d'un dérivé d'indole, soit dans la même composition.

Grâce à l'association avec des sels de terres rares, utilisés conformément à l'invention, on obtient, par ailleurs, après les traitements classiques postérieurs à la teinture, tels que la décoloration, la teinte initiale uniforme du cheveu, ce qui n'est pas possible avec les sels cuivriques, ferreux ou de manganèse de l'art antérieur.

Les teintures ainsi obtenues présentent par ailleurs l'avantage, même lorsqu'elles sont soumises notamment aux agressions atmosphériques (rayons solaires, intempéries, etc...) et aux shampooings, d'être particulièrement homogènes sans présenter les reflets indésirables que l'on observe avec des colorations correspondantes obtenues avec des sels cuivriques, ferreux ou de manganèse, qui peuvent donner lieu à des retours en couleurs hétérogènes et/ou à un virage de teintes vers les couleurs verdâtre, vert bleuté ou orangé.

Par ailleurs, les cheveux sont moins dégradés grâce à l'utilisation de sels de terres rares, notamment des sels de cérium, et présentent un toucher plus doux.

Le procédé et les compositions conformes à l'invention permettent même d'obtenir des colorations plus soutenues, par rapport aux compositions de l'art antérieur ne mettant pas en oeuvre des sels de métaux.

Un objet de l'invention est donc constitué par des procédés de teinture des fibres kératiniques et en particulier des cheveux humains, mettant en oeuvre, dans des étapes séparées, une composition contenant un sel de terre rare et l'application d'une composition contenant au moins un dérivé d'indole de formule (I) définie ci-après.

Un autre objet de l'invention est constitué par un procédé de teinture des fibres kératiniques et en particulier des cheveux humains, mettant en oeuvre dans une seule étape une composition contenant à la fois un sel de terre rare et un dérivé d'indole de formule (I) définie ci-après.

Un autre objet de l'invention est constitué par les compositions mises en oeuvre dans le cadre de ce procédé.

Un autre objet de l'invention est constitué par un dispositif à plusieurs compartiments ou "kit" destiné à être utilisé dans la mise en oeuvre des procédés et des compositions conformes à l'invention.

D'autres objets de l'invention apparaîtront à la lecture de la description et des exemples qui suivent.

Le procédé de teinture des fibres kératiniques et en particulier des cheveux humains, conforme à l'invention, est essentiellement caractérisé par le fait qu'il comprend la mise en oeuvre :

(a) d'un composant (A) contenant dans un milieu approprié pour la teinture, au moins un sel de terre rare,

et

(b) d'un composant (B) contenant dans un milieu approprié pour la teinture, au moins un dérivé d'indole de formule (I) :

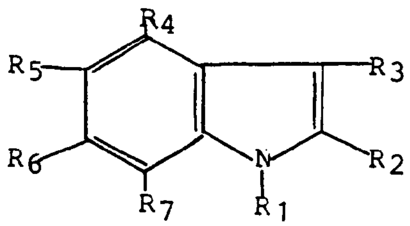

(I)

dans laquelle :

$R_1$ représente un atome d'hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle ou un groupement alcoxy en $C_1$-$C_4$ carbonyle;

$R_4$, $R_5$, $R_5$ et $R_7$, identiques ou différents, représentent un atome d'hydrogène, un radical alkyle en $C_1$-$C_4$, carboxyle, carboxyalkyle en $C_1$-$C_4$, alcoxy $C_1$-$C_4$ carbonyle, alcoxy $C_1$-$C_4$ carbonylalkyle $C_1$-$C_4$, carbamyle, halogène, mono- ou polyhydroxyalkyle en $C_1$-$C_4$, aminoalkyle en $C_1$-$C_4$, un groupement OZ, dans lequel Z désigne hydrogène, alkyle linéaire ou ramifié en $C_1$-$C_{20}$, un groupement aralkyle ($C_1$-$C_4$), un groupenent formyle, un groupement acyle en $C_2$-$C_{20}$ linéaire ou ramifié, un groupement alcénoyle en $C_3$-$C_{20}$ linéaire ou ramifié, un groupement -$SiR_{11}R_{12}R_{13}$, un groupement -$P(O)(OR_8)_2$, un groupement $R_5OSO_2$-; les radicaux $R_4$ et $R_5$, ou bien $R_5$ et $R_6$, ou bien $R_6$ et $R_7$ pouvant former, conjointement avec les atomes de carbone auxquels ils sont rattachés, un cycle contenant éventuellement un groupement carbonyle, un groupement thiocarbonyle, un groupement $>P(O)(OR_8)$ ou un groupement $>CR_9R_{10}$;

sous réserve qu'au moins l'un des radicaux $R_4$ à $R_7$ représente un groupement OZ ou bien que $R_4$ et $R_5$, ou bien $R_5$ et $R_6$, ou bien $R_6$ et $R_7$ forment un cycle, $R_8$ et $R_9$ représentent un atome d'hydrogène ou un groupement alkyle inférieur en $C_1$-$C_4$, $R_{10}$ représente un groupement alcoxy $C_1$-$C_4$ ou un groupement mono- ou dialkyl($C_1$-$C_4$) amino, $R_{11}$, $R_{12}$ et $R_{13}$, identiques ou différents, représentent des groupements alkyle $C_1$-$C_4$, linéaires ou ramifiés,

et les sels correspondants des métaux alcalins, alcalino-terreux, d'ammonium et d'amines, ou les sels d'addition avec les acides minéraux ou organiques,

les composants (A) et (B) étant, soit appliqués sur les fibres à l'aide d'une seule composition, ou bien le composant (A) étant appliqué sur les fibres préalablement ou postérieurement à l'application du composant (B).

Les dérivés d'indole de formule (I) préférés sont choisis parmi le 5,6-dihydroxyindole, le 4-hydroxy 5-méthoxyindole, le 5-méthoxy 6-hydroxyindole, le 2,3-diméthyl 5,6-dihydroxyindole, le 3-méthyl 5,6-dihydroxyindole et le 6-hydroxyindole.

Un dérivé d'indole particulièrement préféré est le 5,6-dihydroxyindole, qui peut être utilisé seul ou en mélange avec les autres dérivés d'indole de formule (I) définis ci-dessus.

Les sels des dérivés d'indole de formule (I) préférés sont choisis parmi les chlorhydrates et les bromhydrates.

Les sels de terres rares utilisés conformément à l'invention sont plus particulièrement choisis parmi les sels de Lanthanides, et notamment parmi les sels de Cérium $Ce^{3+}$, $Ce^{4+}$; de Lanthane $La^{3+}$; d'Europium $Eu^{2+}$, $Eu^{3+}$; de Gadolinium $Gd^{3+}$; d'Ytterbium $Yb^{2+}$, $Yb^{3+}$; de Dysprosium $Dy^{3+}$. Les sels préférés sont en particulier les sulfates, chlorures ou nitrates.

Les sels particulièrement préférés sont les sels de Cérium $Ce^{3+}$ et $Ce^{4+}$ sous la forme de sulfates et de chlorures.

Dans le cadre du procédé en un temps où coexistent le sel de terre rare et le dérivé d'indole de formule (I) définie ci-dessus, les sels préférés sont ceux de Cérium $Ce^{3+}$, d'Europium $Eu^{2+}$, d'Ytterbium $Yb^{2+}$ sous forme de sulfates et de chlorures.

Un premier mode de réalisation de l'invention consiste à appliquer sur les fibres kératiniques, dans un premier temps, le composant (A) sous forme d'une composition contenant dans un milieu approprié pour la teinture, au moins un sel de terre rare, tel que défini ci-dessus.

On fait suivre l'application de cette première composition, après un temps de pose compris entre 1 et 30 minutes et de préférence compris entre 5 et 20 minutes, par l'application du composant (B), constitué par une composition contenant, dans un milieu approprié pour la teinture, le(s) dérivé(s) d'indole de formule (I) définie

ci-dessus.

Une forme de réalisation particulièrement préférée consiste à appliquer dans le premier temps, à titre de sels de terres rares, en particulier lorsque la composition est appliquée sur les cheveux humains, un sel de Cérium $Ce^{3+}$ ou $Ce^{4+}$, et de préférence un sel de $Ce^{3+}$, dans un milieu aqueux approprié pour la teinture, ayant un pH compris entre 2 et 12.

Les cheveux ainsi traités peuvent être rincés à l'eau.

La composition à base de dérivé(s) d'indole appliquée dans le second temps a de préférence un pH compris entre 7,5 et 12 et plus particulièrement entre 8 et 11. Elle est maintenue au contact des fibres pendant un temps de pose compris entre 1 et 30 minutes et de préférence entre 5 et 20 minutes.

Ce traitement peut éventuellement être suivi d'un traitement au peroxyde d'hydrogène à l'aide d'une composition ayant un pH compris entre 2 et 12, ou encore par un traitement avec une base minérale ou organique.

Selon une autre forme de réalisation de l'invention, on peut teindre les cheveux à l'aide d'une composition unique contenant le composant (A) défini ci-dessus et le composant (B). Une telle composition peut éventuellement être préparée juste avant l'emploi.

Des compositions de ce type particulièrement préférées sont des compositions renfermant, à titre de sels de terres rares, du cérium sous forme $Ce^{3+}$ ou $Ce^{4+}$, et en particulier le sel de $Ce^{3+}$ en association avec le(s) dérivé(s) d'indole de formule (I) dans un milieu approprié pour la teinture, à un pH compris entre 2 et 7, et plus particulièrement entre 3 et 6. Le temps de pose est similaire à celui indiqué ci-dessus.

Les compositions prêtes à l'emploi renfermant le sel de $Ce^{3+}$ en association avec un dérivé d'indole de formule (I), dans un milieu approprié pour la teinture, à un pH compris entre 2 et 7, sont particulièrement stables dans le temps.

L'application de cette composition peut éventuellement être suivie d'un traitement au peroxyde d'hydrogène à l'aide d'une composition ayant un pH compris entre 2 et 12 ou par un traitement par une base minérale ou organique.

Les bases minérale ou organique sont choisies plus particulièrement parmi la soude, la potasse, l'ammoniaque et les mono- et triéthanolamines. Les sels de terres rares sont présents dans les compositions utilisées conformément à l'invention, dans des proportions comprises de préférence entre 0,1 et 8% en poids par rapport au poids de la composition totale contenant le sel de terre rare et le dérivé d'indole de formule (I) appliquée sur les fibres. Cette proportion est de préférence comprise entre 0,5 et 5% dans le composant (A).

Le dérivé d'indole de formule (I) est présent de préférence dans des proportions comprises entre 0,1 et 5% en poids et en particulier entre 0,1 et 3% en poids par rapport au poids total de la composition appliquée sur les fibres. Cette proportion est de préférence comprise entre 0,1 et 5% dans le composant (B).

Le milieu approprié pour la teinture, utilisé dans les composants (A) et (B), est de préférence un milieu aqueux constitué par de l'eau ou un mélange d'eau et d'un solvant organique acceptable sur le plan cosmétique, lorsque la composition est destinée à être utilisée dans la teinture des fibres kératiniques humaines.

Les solvants utilisables dans ces compositions sont choisis préférentiellement parmi les alcoolsinférieurs en $C_1$-$C_4$ et plus particulièrement l'alcool éthylique, l'alcool propylique ou isopropylique, l'alcool tertiobutylique, les alkylèneglycols, tels que l'éthylèneglycol, le propylèneglycol, les alkyléthers d'alkylèneglycol, tels que les éthers monométhylique, monoéthylique et monobutylique de l'éthylèneglycol, les monométhyléthers du propylèneglycol et du dipropylèneglycol et le lactate de méthyle.

Les solvants particulièrement préférés sont l'alcool éthylique et le propylèneglycol.

Les compositions renfermant le(s) dérivé(s) d'indole de formule (I) peuvent être stockées en milieu solvant essentiellement anhydre choisi parmi les solvants de préférence indiqués ci-dessus. On appelle un solvant anhydre un solvant contenant moins de 1% d'eau.

Ces compositions sont destinées à être mélangées tout juste avant l'emploi avec un milieu aqueux approprié pour la teinture, tel que défini ci-dessus, ou elles peuvent également être appliquées, directement, au cours du procédé de teinture, sur des cheveux mouillés.

Les compositions utilisées conformément à l'invention peuvent contenir tous autres adjuvants habituellement utilisés en teinture des fibres kératiniques et plus particulièrement des adjuvants cosmétiquement acceptables lorsque ces compositions sont appliquées pour teindre des cheveux humains vivants.

Dans ce dernier cas, les compositions peuvent contenir notamment des amides gras dans des proportions de 0,05 à 10% en poids, des agents tensio-actifs anioniques, cationiques, non ioniques, amphotères ou leurs mélanges, présents de préférence dans des proportions comprises entre 0,1 et 50% en poids, des agents épaississants, des parfums, des agents séquestrants, des agents filmogènes, des agents dispersants, des agents de conditionnement, des agents conservateurs, des agents opacifiants, des agents de gonflement des fibres kératiniques.

Les épaississants sont plus particulièrement choisis parmi l'alginate de sodium, la gomme arabique, la

gomme de guar, les hétérobiopolysaccharides tels que la gomme de xanthane ou les scléroglucanes, les dérivés de cellulose tels que la méthylcellulose, l'hydroxyéthylcellulose, l'hydroxypropylméthylcellulose, le sel de sodium de la carboxyméthylcellulose et des polymères d'acide acrylique préférentiellement réticulés.

On peut également utiliser des agents épaississants minéraux, tels que la bentonite. Ces épaississants sont utilisés seuls ou en mélange et sont présents, de préférence, dans des proportions comprises entre 0,1 et 5% en poids par rapport au poids total de la composition et avantageusement entre 0,5 et 3% en poids.

Les agents alcalinisants utilisables dans les compositions utilisées conformément à l'invention, peuvent être en particulier des amines telles que des alcanolamines, des alkylamines, des hydroxydes ou carbonates alcalins ou d'ammonium. Les agents d'acidification utilisables dans les compositions conformes à l'invention peuvent être choisis parmi l'acide lactique, l'acide acétique, l'acide tartrique, l'acide phosphorique, l'acide chlorhydrique et l'acide citrique. Il est bien entendu possible d'utiliser tout autre agent alcalinisant ou acidifiant acceptable, notamment dans le cas de la teinture des cheveux, en cosmétique.

La composition contenant le(s) dérivé(s) d'indole de formule (I) associé(s) ou non au sel de terre rare, peut être utilisée, dans une forme de réalisation de l'invention, sous forme de mousse aérosol. Elle est conditionnée dans ce cas sous pression dans un dispositif aérosol en présence d'un agent propulseur et d'au moins un générateur de mousse. Des agents générateurs de mousse sont plus particulièrement des polymères moussants, anioniques, cationiques, non ioniques, amphotères ou leurs mélanges, ou des agents tensio-actifs du type de ceux définis ci-dessus.

Une forme de réalisation de l'invention est constituée par une composition à base de dérivé d'indole de formule (I), d'un sel de terre rare dans un milieu approprié pour la teinture, contenant au moins un agent générateur de mousse, conditionnée dans un dispositif aérosol en présence d'un agent propulseur.

La demanderesse a constaté, en particulier, que la mousse produite par expansion à l'air à la sortie du dispositif aérosol est non colorée et qu'elle teint les cheveux rapidement en noir ou dans différents dégradés de gris après un temps de pose de 1 à 30 minutes et en particulier de 1 à 15 minutes.

Le dérivé d'indole de formule (I) associé aux sels de terres rares, tels que $Ce^{3+}$, $Eu^{2+}$, $Yb^{2+}$, peut également être stocké dans un vase clos essentiellement exempt d'oxygène en présence d'un gaz inerte non oxydant tel que l'azote, le dioxyde de carbone, etc.

Dans une autre mise en oeuvre du procédé conforme à l'invention, la composition contenant le(s) dérivé(s) d'indole de formule (I), d'une part, et celle contenant le sel de terre rare, d'autre part, peuvent être conditionnées dans des dispositifs à plusieurs compartiments, encore appelés "kits" ou nécessaires de teinture, comportant tous les composants destinés à être appliqués pour une même teinture sur les fibres kératiniques, en applications successives, avec ou sans prémélange.

Un tel dispositif comporte un premier compartiment contenant, dans un milieu approprié pour la teinture, au moins un sel de terre rare, un second compartiment comprenant une composition contenant, dans un milieu approprié pour la teinture, au moins un dérivé d'indole de formule (I) et éventuellement un troisième compartiment contenant un milieu aqueux approprié pour la teinture et destiné à être mélangé au contenu du deuxième compartiment lorsque la composition contenant le dérivé d'indole est anhydre.

Un dispositif particulièrement approprié pour un mélange extemporané est représenté par un ensemble distributeur du type de celui décrit par la demanderesse dans le brevet français 2.586.913, comportant deux poches séparées réunies par un étui souple. Les deux poches renferment, pour l'une d'entre-elles, au moins le dérivé d'indole dans un milieu approprié pour la teinture, et pour l'autre, au moins le sel de terre rare dans un milieu approprié pour la teinture, défini ci-dessus.

L'application particulièrement préférée du procédé et des compositions conformes à l'invention est la teinture des fibres kératiniques humaines, et en particulier des cheveux humains vivants.

La demanderesse a constaté, par ailleurs, qu'il était possible d'utiliser ces compositions pour la teinture des fourrures ou de la laine.

Les exemples suivants sont destinés à illustrer l'invention sans pour autant présenter un caractère limitatif. Dans les exemples, "MA" signifie Matière Active.

EXEMPLE 1

On procède à la coloration de cheveux naturels gris contenant 90% de blancs, en appliquant successivement deux compositions (A) et (B) et en procédant à un rinçage intermédiaire entre les deux applications.

Composition (A)

| | | |
|---|---|---|
| - $Ce_2(SO_4)_3$, $5H_2O$ | | 2,6 g |
| - Lauryléther sulfate de sodium oxyéthyléné à 2 moles d'oxyde d'éthylène en g MA | | 0,9 g MA |
| - Acide citrique qs pH = 5 | | |
| - Eau | qsp | 100,0 g |

Composition (B)

| | | |
|---|---|---|
| - 5,6-dihydroxyindole | | 1,0 g |
| - Alcool éthylique | | 10,0 g |
| - Lauryléther sulfate de sodium oxyéthyléné à 2 moles d'oxyde d'éthylène en g MA | | 0,9 g MA |
| - NaOH qs pH = 9,5 | | |
| - Eau | qsp | 100,0 g |

On applique la composition (A) du sel de terre rare sur les cheveux, qu'on laisse poser 10 minutes. Après rinçage à l'eau, on applique la composition de 5,6-dihydroxyindole qu'on laisse poser 10 minutes. Après rinçage à l'eau et shampooing, les cheveux sont teints en gris très foncé.

EXEMPLE 2

On procède comme à l'exemple 1 en utilisant une composition de $Ce_2(SO_4)_3$ $5H_2O$ à un pH ajusté à 9,5 par la monoéthanolamine au lieu de 5. On obtient une coloration gris foncé.

EXEMPLE 3

On procède comme à l'exemple 1 en utilisant 1,5 g de $Ce(SO_4)_2$, $3H_2O$ au lieu de 2,6 g de $Ce_2(SO_4)_3$ $5H_2O$. On obtient une coloration gris brun moyen.

EXEMPLE 4

On procède comme à l'exemple 3 en utilisant une composition de $Ce(SO_4)_2$, $3H_2O$ à un pH ajusté à 9,5 par la monoéthanolamine au lieu de 5. On obtient une coloration gris brun moyen légèrement plus soutenu qu'à l'exemple 3.

EXEMPLE 5

On procède à la coloration de cheveux naturels gris contenant 90% de blancs en appliquant une composition renfermant :

| | | |
|---|---|---|
| - 5,6-dihydroxyindole | | 1,0 g |
| - $Ce_2(SO_4)_3$, $5H_2O$ | | 2,6 g |
| - Alcool éthylique | | 10,0 g |
| - Lauryléther sulfate de sodium oxyéthylé à 2 moles d'oxyde d'éthylène en g MA | | 0,9 g MA |
| - Acide citrique qs pH = 5 | | |
| - Eau | qsp | 100,0 g |

On applique la composition ci-dessus sur les cheveux et on la laisse poser 10 minutes. Après rinçage à l'eau et shampooing, on obtient une nuance châtain clair. Après trois applications successives de cette composition, on obtient une nuance châtain foncé.

EXEMPLE 6

On procède à la coloration de cheveux naturels gris contenant 90% de blancs au moyen de la composition suivante conditionnée en kit que l'on prépare au moment de l'emploi par mélange de deux compositions (A) et (B).

Composition (A)

| | | |
|---|---|---|
| - $Ce(SO_4)_2$, $3H_2O$ | | 3,0 g |
| - Lauryléther sulfate de sodium oxyéthylé à 2 moles d'oxyde d'éthylène en g MA | | 1,8 g MA |
| - Acide citrique qs pH = 5 | | |
| - Eau | qsp | 100,0 g |

Composition (B)

| | | |
|---|---|---|
| - 5,6-dihydroxyindole | | 2,0 g |
| - Alcool éthylique | | 20,0 g |
| - Acide citrique qs pH = 5 | | |
| - Eau | qsp | 100,0 g |

Au moment de l'emploi, on effectue un mélange en poids 50/50 des deux compositions (A) + (B). On applique la composition résultante sur les cheveux pendant 10 minutes. On rince à l'eau. Après shampooing, les cheveux sont teints en châtain. Quatre applications successives de cette composition donnent une coloration châtain foncé.

EXEMPLE 7

On procède comme à l'exemple 6 en ajustant le pH des deux compositions à 9,5 avec de la monoéthanolamine. On obtient une coloration blond foncé après quatre applications successives de la composition.

EXEMPLE 8

Sur des cheveux colorés par une application de la composition de l'exemple 5, on effectue un traitement oxydant d'une durée de 10 minutes avec une solution aqueuse de peroxyde d'hydrogène à 10 volumes à pH spontané 3,95. On obtient après rinçage à l'eau une coloration gris foncé.

EXEMPLE 9

Sur des cheveux colorés par une application de la composition de l'exemple 6, on effectue un traitement oxydant d'une durée de 10 minutes avec une solution aqueuse de peroxyde d'hydrogène à 10 volumes à pH spontané 3,95. Après rinçage à l'eau, les cheveux sont teints en gris brun foncé.

EXEMPLE 10

On procède à la coloration de cheveux naturels gris à 90% de blancs en appliquant une composition prête à l'emploi, renfermant :

| | | |
|---|---|---|
| - 5,6-dihydroxyindole | | 0,15 g |
| - $CeCl_3$, $7H_2O$ | | 1,0 g |
| - Gomme de guar vendue sous la dénomination JAGUAR HP 60 par la Société CELANESE | | 1,0 g |
| - Alcool éthylique | | 10,0 g |
| - pH spontané = 5 | | |
| - Eau | qsp | 100,0 g |

On applique la composition ci-dessus sur les cheveux et on la laisse poser 10 minutes. Après rinçage à l'eau et séchage, on obtient une nuance gris cendré moyen.

EXEMPLE 11

On procède à la coloration de cheveux naturels gris contenant 90% de blancs, au moyen de la composition suivante conditionnée en kit, que l'on prépare au moment de l'emploi par mélange de deux compositions (A) et (B) :

Composition (A)

| | | |
|---|---|---|
| - $CeCl_3$, $7H_2O$ | | 1,0 g |
| - Acide citrique qs pH = 5 | | |
| - Eau | qsp | 100,0 g |

Composition (B)

| | | | |
|---|---|---|---|
| - 5,6-dihydroxyindole | | 2,0 | g |
| - Alcool éthylique | | 20,0 | g |
| - Lauryléther sulfate de sodium oxyéthyléné à 2 moles d'oxyde d'éthylène en g MA | | 1,8 | g MA |
| - Acide citrique qs pH = 5 | | | |
| - Eau | qsp | 100,0 | g |

Au moment de l'emploi, on mélange les deux compositions (A) et (B) dans un rappport pondéral 50/50. On applique la composition résultante sur les cheveux pendant 10 minutes. On rince à l'eau. Après shampooing, les cheveux sont teints en gris foncé.

EXEMPLE 12

On procède à la coloration de cheveux naturels gris contenant 90% de blancs, en appliquant successivement deux compositions (B) puis (A) sans procéder à un rinçage intermédiaire.

Composition (A)

| | | | |
|---|---|---|---|
| - $CeCl_3$, $7H_2O$ | | 1,5 | g |
| - Lauryléther sulfate de sodium oxyéthyléné à 2 moles d'oxyde d'éthylène en g MA | | 0,9 | g MA |
| - Monoéthanolamine qs pH = 6 | | | |
| - Eau | qsp | 100,0 | g |

Composition (B)

| | | | |
|---|---|---|---|
| - 5,6-dihydroxyindole | | 1,0 | g |
| - Alcool éthylique | | 10,0 | g |
| - Lauryléther sulfate de sodium oxyéthyléné à 2 moles d'oxyde d'éthylène en g MA | | 0,9 | g MA |
| - NaOH qs pH = 9,5 | | | |
| - Eau | qsp | 100,0 | g |

On applique la composition (B) de 5,6-dihydroxyindole sur les cheveux et on la laisse poser 10 minutes.
On applique ensuite la composition du sel de terre rare (A) qu'on laisse poser 10 minutes.
Après rinçage à l'eau et shampooing, les cheveux sont teints en gris foncé.

EXEMPLE 13

On procède à la coloration de cheveux naturels gris à 90% de blancs, en appliquant une composition prête à l'emploi, renfermant :

| | | |
|---|---|---|
| - 5,6-dihydroxyindole | | 0,75 g |
| - $CeCl_3$, $7H_2O$ | | 3,7 g |
| - pH spontané = 4,47 | | |
| - Eau | qsp | 100,0 g |

On applique la composition ci-dessus sur les cheveux et on la laisse poser 10 minutes.
Après rinçage à l'eau et séchage, on obtient une nuance gris foncé.

EXEMPLE 14

On procède à la coloration de cheveux permanentés gris à 90% de blancs, en appliquant une composition prête à l'emploi, renfermant :

| | | |
|---|---|---|
| - 4-hydroxy 5-méthoxyindole | | 0,5 g |
| - $Ce_2(SO_4)_3$, $5H_2O$ | | 0,2 g |
| - Alcool éthylique | | 16,5 g |
| - pH spontané = 3,9 | | |
| - Eau | qsp | 100,0 g |

On applique la composition ci-dessus sur les cheveux et on la laisse poser 10 minutes. Après rinçage à l'eau, shampooing et séchage, on obtient une nuance gris pourpre.

EXEMPLE 15

On reproduit l'exemple 14 en faisant suivre les 10 minutes de pose de la composition par un essorage des mèches et l'application d'une solution aqueuse alcaline de NaOH à pH 11,9 pendant 10 minutes. Après rinçage à l'eau, shampooing et séchage, on obtient une nuance pourpre.

EXEMPLE 16

On procède à la coloration de cheveux permanentés, gris à 90% de blancs, en appliquant une composition prête à l'emploi, renfermant :

| | | | |
|---|---|---|---|
| - 5,6-dihydroxyindole | | 0,5 | g |
| - Bromhydrate de 2,3-diméthyl 5,6-dihydroxyindole | | 1,0 | g |
| - Alcool éthylique | | 10,0 | g |
| - Gomme de guar vendue sous la dénomination JAGUAR HP60 par la Société CELANESE | | 1,0 | g |
| - Alkyléther de glycoside vendu à la concentration de 60% MA sous la dénomination TRITON CG 110 par la Société SEPPIC | | 5,0 | g MA |
| - Conservateurs | qs | | |
| - Eau | qsp | 80,0 | g |
| - pH ajusté à 7,6 par de la triéthanolamine | | | |

à cette solution on ajoute 20 g de de $CeCl_3$, $7H_2O$ en solution aqueuse à 5% (soit 1 g MA en $CeCl_3$, $7H_2O$

Le pH final est égal à 6,5

On applique la composition ci-dessus sur les cheveux et on la laisse poser 15 minutes. Après rinçage à l'eau, shampooing et séchage, on obtient une nuance gris légèrement nacré. Une nouvelle application de la composition de la même durée donne, après rinçage à l'eau, shampooing et séchage, une nuance acajou.

EXEMPLE 17

On procède à la coloration de cheveux naturels gris contenant 90% de blancs, au moyen de la composition suivante conditionnée en kit, que l'on prépare au moment de l'emploi par mélange de deux compositions (A) et (B).

Composition (A)

| | | | |
|---|---|---|---|
| - $Ce(SO_4)_2$, $3H_2O$ | | 0,8 | g |
| - pH spontané = 2 | | | |
| - Eau | qsp | 100,0 | g |

Composition (B)

| | | | |
|---|---|---|---|
| - 3-méthyl 5,6-dihydroxyindole | | 4,0 | g |
| - Alcool éthylique | qsp | 100,0 | g |

Au moment de l'emploi, on effectue un mélange en poids 50/50 des deux compositions (A) et (B). On applique la composition résultante sur les cheveux pendant 15 minutes. On rince à l'eau. Après shampooing et séchage, les cheveux sont teints uniformément en gris.

EXEMPLE 18

On procède à la coloration de cheveux permanentés gris contenant 90% de blancs, en appliquant successivement deux compositions (A) et (B).

Composition (A)

| | | | |
|---|---|---|---|
| - $CeCl_3$, $7H_2O$ | | 1,0 | g |
| - pH spontané = 5,4 | | | |
| - Eau | qsp | 100,0 | g |

Composition (B)

| | | | |
|---|---|---|---|
| - 5-méthoxy 6-hydroxyindole | | 1,0 | g |
| - Alcool éthylique | | 10,0 | g |
| - pH spontané = 8 | | | |
| - Eau | qsp | 100,0 | g |

On applique la composition (A) du sel de terre rare sur les cheveux, qu'on laisse poser 5 minutes. Après essorage, on applique la composition (B) qu'on laisse poser 10 minutes. Après rinçage à l'eau, shampooing et séchage, les cheveux sont teints uniformément en gris.

EXEMPLE 19

On procède à la coloration de cheveux naturels gris contenant 90% de blancs, en appliquant successivement deux compositions (B) puis (A).

Composition (A)

| | | | |
|---|---|---|---|
| - $Ce_2(SO_4)_3$, $5H_2O$ | | 1,25 | g |
| - pH spontané = 2 | | | |
| - Eau | qsp | 100,0 | g |

Composition (B)

| | | |
|---|---|---|
| - 6-hydroxyindole | | 1,0 g |
| - Alcool éthylique | | 25,0 g |
| - pH spontané = 6 | | |
| - Eau | qsp | 100,0 g |

On applique la composition (B) du dérivé indolique sur les cheveux, qu'on laisse poser 10 minutes. Après essorage, on applique la composition (A) de sel de terre rare qu'on laisse poser 10 minutes. Après rinçage à l'eau, shampooing et séchage, les cheveux sont teints uniformément en gris clair nacré.

EXEMPLE 20

On procède à la coloration de cheveux naturels gris contenant 90% de blancs, en appliquant successivement deux compositions (B) puis (A) et en procédant à un rinçage intermédiaire entre les deux applications.

Composition (A)

| | | |
|---|---|---|
| - $Ce_2(SO_4)_3$, $5H_2O$ | | 1,5 g |
| - pH spontané = 2 | | |
| - Eau | qsp | 100,0 g |

Composition (B)

| | | |
|---|---|---|
| - 6-hydroxyindole | | 1,0 g |
| - 5,6-dihydroxyindole | | 0,7 g |
| - Alcool éthylique | | 16,5 g |
| - pH spontané = 7,2 | | |
| - Eau | qsp | 100,0 g |

On applique la composition (B) de dérivé indolique sur les cheveux, qu'on laisse poser 30 minutes. Après rinçage à l'eau, on applique la composition (A) de sel de terre rare qu'on laisse poser 10 minutes. Après rinçage, shampooing puis séchage, les cheveux sont teints uniformément en gris nacré.

**Revendications**

**1.** Procédé de teinture des fibres kératiniques, caractérisé par le fait qu'il comprend l'application :
(a) d'un composant (A) contenant, dans un milieu approprié pour la teinture, au moins un sel de terre rare, et
(b) d'un composant (B) contenant, dans un milieu approprié pour la teinture, au moins un dérivé d'indole de formule (I) :

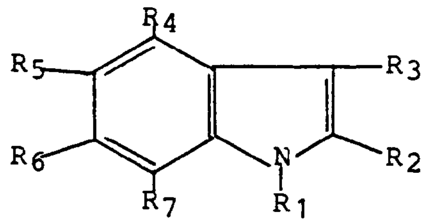

(I)

dans laquelle :

$R_1$ représente un atome d'hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle ou un groupement alcoxy en $C_1$-$C_4$ carbonyle;

$R_4$, $R_5$, $R_6$ et $R_7$, identiques ou différents, représentent un atome d'hydrogène, un radical alkyle en $C_1$-$C_4$, carboxyle, carboxyalkyle en $C_1$-$C_4$, alcoxy $C_1$-$C_4$ carbonyle, alcoxy $C_1$-$C_4$ carbonylalkyle $C_1$-$C_4$, carbamyle, halogène, mono- ou polyhydroxyalkyle en $C_1$-$C_4$, aminoalkyle en $C_1$-$C_4$, un groupement OZ, dans lequel Z désigne hydrogène, alkyle linéaire ou ramifié en $C_1$-$C_{20}$, un groupement aralkyle ($C_1$-$C_4$), un groupement formyle, un groupenent acyle en $C_2$-$C_{20}$ linéaire ou ramifié, un groupement alcénoyle en $C_3$-$C_{20}$ linéaire ou ramifié, un groupement $-SiR_{11}R_{12}R_{13}$, un groupement $-P(O)(OR_6)_2$, un groupement $R_6OSO_2$-; les radicaux $R_4$ et $R_6$, ou bien $R_6$ et $R_6$, ou bien $R_6$ et $R_7$ pouvant former, conjointement avec les atomes de carbone auxquels ils sont rattachés, un cycle contenant éventuellement un groupement carbonyle, un groupement thiocarbonyle, un groupement $>P(O)(OR_8)$ ou un groupement $>CR_9R_{10}$;

sous réserve qu'au moins l'un des radicaux $R_4$ à $R_7$ représente un groupement OZ ou bien que $R_4$ et $R_6$, ou bien $R_6$ et $R_6$, ou bien $R_6$ et $R_7$ forment un cycle, $R_8$ et $R_9$ représentent un atome d'hydrogène ou un groupement alkyle inférieur en $C_1$-$C_4$, $R_{10}$ représente un groupement alcoxy $C_1$-$C_4$ ou un groupement mono- ou dialkyl($C_1$-$C_4$) amino, $R_{11}$, $R_{12}$ et $R_{13}$, identiques ou différents, représentent des groupements alkyle $C_1$-$C_4$, linéaires ou ramifiés,

et les sels correspondants des métaux alcalins, alcalino-terreux, d'ammonium et d'amines, ou les sels d'addition avec des acides minéraux ou organiques,

les composants (A) et (B) étant appliqués sur les fibres à l'aide d'une seule composition, ou le composant (A) étant appliqué sur les fibres préalablement ou postérieurement à l'application du composant (B).

**2.** Procédé selon la revendication 1, caractérisé par le fait que le composant (B) contient du 5,6-dihydroxyindole.

**3.** Procédé selon la revendication 1, caractérisé par le fait que le composant (B) contient un composé choisi parmi le 4-hydroxy 5-méthoxyindole, le 5-méthoxy 6-hydroxyindole, le 2,3-diméthyl 5,6-dihydroxy indole, le 3-méthyl 5,6-dihydroxyindole et le 6-hydroxyindole.

**4.** Procédé selon les revendications 1 à 3, caractérisé par le fait que l'on applique dans un premier temps une composition constituée par le composant (A) et qu'après un temps de pose de 1 à 30 minutes, on applique la composition constituée par le composant (B) contenant au moins un dérivé d'indole de formule (I) définie dans la revendication 1.

**5.** Procédé selon l'une des revendications 1 à 4, caractérisé par le fait que l'application des deux compositions (A) et (B) est suivie par un traitement au peroxyde d'hydrogène ou avec une base minérale ou organique.

**6.** Procédé selon l'une quelconque des revendications 1 à 5, caractérisé par le fait que l'application des composants (A) et (B) est séparée par une étape de rinçage à l'eau des fibres traitées.

**7.** Procédé selon l'une quelconque des revendications 1 à 6, caractérisé par le fait que les sels de terres rares sont choisis parmi les sels de lanthanides.

**8.** Procédé selon l'une quelconque des revendications 1 à 7, caractérisé par le fait que les sels de terres rares sont choisis parmi les sels de Cérium $Ce^{3+}$, $Ce^{4+}$; de Lanthane $La^{3+}$; d'Europium $Eu^{2+}$, $Eu^{3+}$; de Gadolinium $Gd^{3+}$; d'Ytterbium $Yb^{2+}$, $Yb^{3+}$; de Dysprosium $Dy^{3+}$.

**9.** Procédé selon l'une quelconque des revendications 1 à 8, caractérisé par le fait que les sels de terres rares sont choisis parmi les sulfates, chlorures et nitrates de terres rares.

**10.** Procédé selon l'une quelconque des revendications 1 à 9, caractérisé par le fait que l'on applique sur les fibres une composition (A) contenant dans un milieu approprié pour la teinture, un sel de Cérium $Ce^{3+}$ ou $Ce^{4+}$ ayant un pH compris entre 2 et 12 et que l'on fait suivre ou précéder cette application par l'application d'une solution contenant au moins un dérivé d'indole de formule (I) dans un milieu approprié pour la teinture, ayant un pH compris entre 7,5 et 12 et de préférence entre 8 et 11.

**11.** Procédé selon la revendication 10, caractérisé par le fait que l'on fait suivre l'application des deux compositions par un traitement avec une solution de peroxyde d'hydrogène ayant un pH compris entre 2 et 12 ou par un traitement avec une base minérale ou organique.

**12.** Procédé selon la revendication 11, caractérisé par le fait qu'il comprend l'application d'une seule composition contenant le composant (A) et le composant (B) pendant un temps de pose compris entre 1 et 30 minutes et de préférence entre 1 et 15 minutes, et que l'on procède ensuite au rinçage à l'eau.

**13.** Procédé selon la revendication 12, caractérisé par le fait que l'on mélange de façon extemporanée les composants (A) et (B) tout juste avant la teinture des fibres et qu'on procède, ensuite, à l'application de la composition dans des quantités efficaces pour teindre les fibres kératiniques.

**14.** Procédé selon l'une quelconque des revendications 1,2,3,7,8,12 ou 13, caractérisé par le fait que le sel de terre rare est choisi parmi le sel de Cérium $Ce^{3+}$, le sel d'Europium $Eu^{2+}$ ou le sel d'Ytterbium $Yb^{2+}$, sous forme de sulfate ou de chlorure.

**15.** Procédé selon l'une quelconque des revendications 1,2,3,7,8,12 à 14, caractérisé par le fait que l'on applique sur les fibres une composition unique, renfermant à titre de composant (A) un sulfate ou chlorure de $Ce^{3+}$ et à titre de composant (B), au moins un dérivé d'indole de formule (I), à un pH compris entre 2 et 7 et de préférence entre 3 et 6.

**16.** Procédé selon la revendication 15, caractérisé par le fait qu'on fait suivre l'application par un traitement au peroxyde d'hydrogène à un pH compris entre 2 et 12 ou par un traitement avec une base minérale ou organique.

**17.** Procédé selon l'une quelconque des revendications 1 à 16, caractérisé par le fait que l'on teint des cheveux humains.

**18.** Composition destinée à être utilisée pour la teinture des fibres kératiniques, caractérisée par le fait qu'elle contient, dans un milieu approprié pour la teinture, au moins un sel de terre rare et au moins un dérivé d'indole de formule (I) :

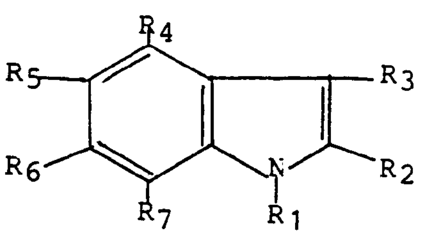

(I)

dans laquelle :

$R_1$ représente un atome d'hydrogène ou un groupement alkyle en $C_1$-$C_4$;

$R_2$ et $R_3$, identiques ou différents, représentent un atome d'hydrogène, un groupement alkyle en $C_1$-$C_4$, un groupement carboxyle ou un groupement alcoxy en $C_1$-$C_4$ carbonyle;

$R_4$, $R_5$, $R_6$ et $R_7$, identiques ou différents, représentent un atome d'hydrogène, un radical alkyle en $C_1$-$C_4$, carboxyle, carboxyalkyle en $C_1$-$C_4$, alcoxy $C_1$-$C_4$ carbonyle, alcoxy $C_1$-$C_4$ carbonylalkyle $C_1$-$C_4$, carbamyle, halogène, mono- ou polyhydroxyalkyle en $C_1$-$C_4$, aminoalkyle en $C_1$-$C_4$, un groupement OZ, dans lequel Z désigne hydrogène, alkyle linéaire ou ramifié en $C_1$-$C_{20}$, un groupement aralkyle ($C_1$-$C_4$), un groupement formyle, un groupement acyle en $C_2$-$C_{20}$ linéaire ou ramifié, un groupement alcénoyle en $C_3$-$C_{20}$

linéaire ou ramifié, un groupement $-SiR_{11}R_{12}R_{13}$, un groupement $-P(O)(OR_8)_2$, un groupement $R_8OSO_2$-; les radicaux $R_4$ et $R_8$, ou bien $R_8$ et $R_8$, ou bien $R_8$ et $R_7$ pouvant former, conjointement avec les atomes de carbone auxquels ils sont rattachés, un cycle contenant éventuellement un groupement carbonyle, un groupement thiocarbonyle, un groupement $>P(O)(OR_8)$ ou un groupement $>CR_9R_{10}$;

sous réserve qu'au moins l'un des radicaux $R_4$ à $R_7$ représente un groupement OZ ou bien que $R_4$ et $R_5$ ou bien $R_5$ et $R_8$, ou bien $R_6$ et $R_7$ forment un cycle, $R_8$ et $R_9$ représentent un atome d'hydrogène ou un groupement alkyle inférieur en $C_1$-$C_4$, $R_{10}$ représente un groupement alcoxy $C_1$-$C_4$ ou un groupement mono- ou dialkyl($C_1$-$C_4$) amino, $R_{11}$ $R_{12}$ et $R_{13}$, identiques ou différents, représentent des groupements alkyle $C_1$-$C_4$, linéaires ou ramifiés,

et les sels correspondants des métaux alcalins, alcalino-terreux, d'ammonium et d'amines, ou les sels d'addition avec les acides minéraux ou organiques.

**19.** Composition selon la revendication 18, caractérisée par le fait que le dérivé d'indole est le 5,6-dihydroxyindole.

**20.** Composition selon la revendication 18, caractérisée par le fait que le dérivé d'indole est choisi parmi le 4-hydroxy 5-méthoxyindole, le 5-méthoxy 6-hydroxyindole, le 2,3-diméthyl 5,6-dihydroxyindole, le 3-méthyl 5,6-dihydroxyindole et le 6-hydroxyindole.

**21.** Composition selon les revendications 18 à 20, caractérisée par le fait que le sel de terre rare est présent dans des proportions comprises entre 0,1 et 8% en poids par rapport au poids total de la composition.

**22.** Composition selon les revendications 18 à 20, caractérisée par le fait que le dérivé d'indole de formule (I) est présent dans des proportions comprises entre 0,1 et 5% en poids et de préférence entre 0,1 et 3% en poids par rapport au poids total de la composition appliquée sur les fibres.

**23.** Composition selon l'une quelconque des revendications 18 à 22, caractérisée par le fait que le milieu approprié pour la teinture est constitué par un milieu aqueux formé par de l'eau ou par un mélange eau/solvant organique.

**24.** Composition selon l'une quelconque des revendications 18 à 23, caractérisée par le fait que le milieu approprié pour la teinture est constitué par un milieu solvant essentiellement anhydre.

**25.** Composition selon l'une quelconque des revendications 18 à 24, caractérisée par le fait que les solvants sont choisis parmi l'alcool éthylique, l'alcool propylique ou isopropylique, l'alcool tertiobutylique, l'éthylèneglycol, le propylèneglycol, les éthers monométhylique, monoéthylique et monobutylique de l'éthylèneglycol, les monométhyléthers du propylèneglycol et du dipropylèneglycol, le lactate de méthyle.

**26.** Composition selon l'une quelconque des revendications 18 à 25, caractérisée par le fait qu'elle est conditionnée dans un vase clos essentiellement exempt d'oxygène et en présence d'un gaz inerte non oxydant.

**27.** Composition selon l'une quelconque des revendications 18 à 26, caractérisée par le fait qu'elle est conditionnée dans un dispositif aérosol en présence d'un gaz propulseur et d'un agent générateur de mousse.

**28.** Composition selon l'une quelconque des revendications 18 à 27, caractérisée par le fait qu'elle contient un ou plusieurs adjuvants choisis parmi les amides gras dans des proportions de 0,05 à 10%, les agents tensio-actifs anioniques, cationiques, non ioniques, amphotères ou leurs mélanges, présents dans des proportions comprises entre 0,1 et 50%, les agents épaississants présents dans des proportions comprises entre 0,1 et 5%, les parfums, les agents séquestrants, les agents filmogènes, les agents dispersants, les agents de conditionnement, les agents conservateurs, les agents opacifiants, des agents de gonflement des fibres kératiniques.

**29.** Dispositif à plusieurs compartiments ou "kit" destiné à être utilisé en teinture des fibres kératiniques et plus particulièrement des cheveux humains, caractérisé par le fait qu'il comprend, dans un premier compartiment, un composant (A) contenant dans un milieu approprié pour la teinture au moins un sel de terre rare; dans un second compartiment, un composant (B) contenant dans un milieu approprié pour la teinture, au moins un dérivé d'indole de formule (I)

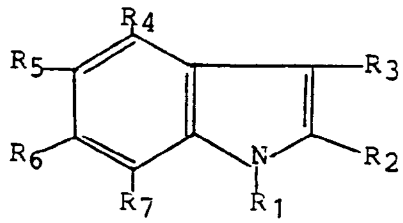

(I)

dans laquelle :

$R_1$ représente un atome d'hydrogène ou un groupement alkyle en $C_1$-$C_4$;

$R_2$ et $R_3$, identiques ou différents, représentent un atome d'hydrogène, un groupenent alkyle en $C_1$-$C_4$, un groupement carboxyle ou un groupement alcoxy en $C_1$-$C_4$ carbonyle;

$R_4$, $R_5$, $R_6$ et $R_7$, identiques ou différents, représentent un atome d'hydrogène, un radical alkyle en $C_1$-$C_4$, carboxyle, carboxyalkyle en $C_1$-$C_4$, alcoxy $C_1$-$C_4$ carbonyle, alcoxy $C_1$-$C_4$ carbonylalkyle $C_1$-$C_4$, carbamyle, halogène, mono- ou polyhydroxyalkyle en $C_1$-$C_4$, aminoalkyle en $C_1$-$C_4$, un groupement OZ, dans lequel Z désigne hydrogène, alkyle linéaire ou ramifié en $C_1$-$C_{20}$, un groupement aralkyle ($C_1$-$C_4$), un groupement formyle, un groupement acyle en $C_2$-$C_{20}$ linéaire ou ramifié, un groupement alcénoyle en $C_3$-$C_{20}$ linéaire ou ramifié, un groupement $-SiR_{11}R_{12}R_{13}$, un groupement $-P(O)(OR_6)_2$, un groupement $R_6OSO_2$-; les radicaux $R_4$ et $R_6$, ou bien $R_6$ et $R_6$, ou bien $R_6$ et $R_7$ pouvant former, conjointement avec les atomes de carbone auxquels ils sont rattachés, un cycle contenant éventuellement un groupement carbonyle, un groupement thiocarbonyle, un groupement $>P(O)(OR_8)$ ou un groupement $>CR_9R_{10}$;

sous réserve qu'au moins l'un des radicaux $R_4$ à $R_7$ représente un groupement OZ ou bien que $R_4$ et $R_6$, ou bien $R_6$ et $R_6$, ou bien $R_6$ et $R_7$ forment un cycle, $R_8$ et $R_9$ représentent un atome d'hydrogène ou un groupement alkyle inférieur en $C_1$-$C_4$, $R_{10}$ représente un groupement alcoxy $C_1$-$C_4$ ou un groupement mono- ou dialkyl($C_1$-$C_4$) amino, $R_{11}$, $R_{12}$ et $R_{13}$, identiques ou différents, représentent des groupements alkyle $C_1$-$C_4$, linéaires ou ramifiés,

et les sels correspondants des métaux alcalins, alcalino-terreux, d'ammonium et d'amines, ou les sels d'addition avec des acides minéraux ou organiques.

**30.** Dispositif selon la revendication 29, caractérisé par le fait qu'il comprend un troisième compartiment contenant un milieu aqueux approprié pour la teinture et destiné à être mélangé au contenu du second compartiment lorsque le milieu approprié pour la teinture, de la composition présente dans ce second compartiment, est anhydre.

**Patentansprüche**

**1.** Verfahren zur Färbung keratinischer Fasern, dadurch **gekennzeichnet,** daß man aufbringt:

(a) einen Bestandteil (A), enthaltend, in einem zur Färbung geeigneten Milieu, mindestens ein Salz eines seltenen Erdmetalls, und

(b) einen Bestandteil (B), enthaltend, in einem zur Färbung geeigneten Milieu, mindestens ein Indolderivat der Formel (I):

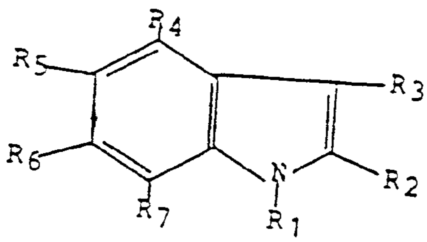

(I)

worin:

$R_1$ ein Wasserstoffatom oder eine $C_{1-4}$-Alkylgruppe darstellt;

$R_2$ und $R_3$, gleich oder verschieden, ein Wasserstoffatom, eine $C_{1-4}$-Alkyl-, Carboxyl- oder $C_{1-4}$-Alkoxycarbonylgruppe darstellen;

$R_4$, $R_5$, $R_6$ und $R_7$, gleich oder verschieden, ein Wasserstoffatom, einen $C_{1-4}$-Alkyl-, Carboxyl-, Carboxy-$C_{1-4}$-alkyl-, $C_{1-4}$-Alkoxycarbonyl-,

$C_{1-4}$-Akoxycarbonyl-$C_{1-4}$-alkyl-, Carbamyl-, Halogen-, Mono- oder Polyhydroxy-$C_{1-4}$-alkyl-, $C_{1-4}$-Aminoalkylrest, eine Gruppe OZ darstellen, worin Z Wasserstoff, einen linearen oder verzweigten $C_{1-20}$-Alkylrest, eine Ar-$C_{1-4}$-alkyl-, Formyl-, lineare oder verzweigte $C_{2-20}$-Acyl-, lineare oder verzweigte $C_{3-20}$-Alkenoyl-, eine -$SiR_{11}R_{12}R_{13}$-, -$P(O)(OR_8)_2$-, $R_8OSO_2$-Gruppe bedeutet, wobei die Reste $R_4$ und $R_5$ oder auch $R_5$ und $R_6$ oder auch $R_6$ und $R_7$ zusammen mit den Kohlenstoffatomen, an die sie gebunden sind, einen Ring bilden können, der gegebenenfalls eine Carbonyl-, Thiocabonyl, $>P(O)(OR_8)$- oder $>CR_9R_{10}$-Gruppe enthält,

mit der Maßgabe, daß mindestens einer der Reste $R_4$ bis $R_7$ eine Gruppe OZ darstellt, oder daß $R_4$ und $R_5$ oder auch $R_5$ und $R_6$ oder auch $R_6$ und $R_7$ einen Ring bilden, wobei $R_5$ und $R_9$ ein Wasserstoffatom oder eine $C_{1-4}$- Niedrigalkylgruppe darstellen, $R_{10}$ eine $C_{1-4}$-Alkoxygruppe oder eine Mono- oder Di-$C_{1-4}$-alkylaminogruppe darstellt, $R_{11}$, $R_{12}$, $R_{13}$, gleich oder verschieden, lineare oder verzweigte $C_{1-4}$-Alkylgruppen darstellen,

und die entsprechenden Salze von Alkali-, Erdalkalimetallen, von Ammonium und von Aminen, oder die Additionssalze mit Mineralsäuren oder organischen Säuren,

wobei die Bestandteile (A) und (B) auf die Fasern mit einer einzigen Zusammensetzung oder der Bestandteil (A) auf die Fasern vor oder nach der Aufbringung des Bestandteils (B) aufgebracht werden.

2. Verfahren gemäß Anspruch 1,
dadurch **gekennzeichnet,** daß der Bestandteil (B) 5,6-Dihydroxyindol enthält.

3. Verfahren gemäß Anspruch 1,
dadurch **gekennzeichnet,** daß
der Bestandteil (B) eine Verbindung enthält, ausgewählt aus 4-Hydroxy-5-methoxyindol, 5-Methoxy-6-hydroxyindol, 2,3-Dimethyl-5,6-dihydroxyindol, 3-Methyl-5,6-dihydroxyindol und 6-Hydroxyindol.

4. Verfahren gemäß Ansprüchen 1 bis 3,
dadurch **gekennzeichnet,** daß man zuerst eine Zusammensetzung aus dem Bestandteil (A) und nach einer Verweilzeit von 1 bis 30 Minuten die Zusammensetzung aus dem Bestandteil (B), enthaltend mindestens ein in Anspruch 1 definiertes Indolderivat der Formel (I), aufbringt.

5. Verfahren gemäß einem der Ansprüche 1 bis 4,
dadurch **gekennzeichnet,** daß nach der Aufbringung der beiden Zusammensetzungen (A) und (B) eine Behandlung mit Wasserstoffperoxid oder mit einer Mineralbase oder einer organischen Base erfolgt.

6. Verfahren gemäß jedem Anspruch 1 bis 5,
dadurch **gekennzeichnet,** daß die Aufbringung der Bestandteile (A) und (B) durch eine Stufe getrennt ist, in der man die behandelten Fasern mit Wasser spült.

7. Verfahren gemäß jedem Anspruch 1 bis 6,
dadurch **gekennzeichnet,** daß die Salze der seltenen Erden ausgewählt sind aus Lanthanidensalzen.

8. Verfahren gemäß jedem Anspruch 1 bis 7,
dadurch **gekennzeichnet,** daß die Salze der seltenen Erden ausgewählt sind unter den Cersalzen $Ce^{3+}$, $Ce^{4+}$, Lanthansalzen $La^{3+}$, Europiumsalzen $Eu^{2+}$, $Eu^{3+}$, Gadoliniumsalzen $Gd^{3+}$, Ytterbiumsalzen $Yb^{2+}$, $Yb^{3+}$ und Dysprosiumsalzen $Dy^{3+}$.

9. Verfahren gemäß jedem Anspruch 1 bis 8,
dadurch **gekennzeichnet,** daß die Salze der seltenen Erden ausgewählt sind aus Sulfaten, Chloriden und Nitraten von seltenen Erden.

10. Verfahren gemäß jedem Anspruch 1 bis 9,
dadurch **gekennzeichnet,** daß man auf die Fasern eine Zusammensetzung (A), enthaltend in einem zur Färbung geeigneten Milieu ein Cersalz $Ce^{3+}$ oder $Ce^{4+}$ mit einem pH von 2 bis 12, und vor oder nach dieser Aufbringung eine Lösung aufbringt, enthaltend mindestens ein Indolderivat der Formel (I) in einem zur Färbung geeigneten Milieu mit einem pH von 7,5 bis 12 und vorzugsweise von 8 bis 11.

11. Verfahren gemäß Anspruch 10,

dadurch **gekennzeichnet,** daß man nach der Aufbringung der beiden Zusammensetzungen eine Behandlung mit einer Lösung von Wasserstoffperoxid mit einem pH von 2 bis 12 oder eine Behandlung mit einer Mineralbase oder einer organischen Base folgen läßt.

**12.** Verfahren gemäß Anspruch 11,
dadurch **gekennzeichnet,** daß man eine einzige Zusammensetzung, enthaltend den Bestandteil (A) und (B), während einer Verweilzeit von 1 bis 30 Minuten und vorzugsweise von 1 bis 15 Minuten aufbringt und dann mit Wasser spült.

**13.** Verfahren gemäß Anspruch 12,
dadurch **gekennzeichnet,** daß man unmittelbar die Bestandteile (A) und (B) ganz kurz vor der Färbung der Fasern vermischt und anschließend die Zusammensetzung in zur Färbung der keratinischen Fasern wirksamen Mengen aufbringt.

**14.** Verfahren gemäß jedem Anspruch 1, 2, 3, 7, 8, 12 oder 13,
dadurch **gekennzeichnet,** daß das Salz der seltenen Erde ausgewählt ist aus dem Salz von Cer $Ce^{3+}$, dem Salz von Europium $Eu^{2+}$ oder dem Salz von Ytterbium $Yb^{2+}$, und zwar in der Form von Sulfat oder Chlorid.

**15.** Verfahren gemäß jedem Anspruch 1, 2, 3, 7, 8, 12 bis 14,
dadurch **gekennzeichnet,** daß man auf die Fasern eine einzige Zusammensetzung, umfassend als Bestandteil (A) ein Sulfat oder Chlorid von $Ce^{3+}$ und als Bestandteil (B) mindestens ein Indolderivat der Formel (I) bei einem pH von 2 bis 7 und vorzugsweise von 3 bis 6 aufbringt.

**16.** Verfahren gemäß Anspruch 15,
dadurch **gekennzeichnet,** daß man nach der Aufbringung eine Behandlung mit Wasserstoffperoxid bei einem pH von 2 bis 12 oder eine Behandlung mit einer Mineralbase oder einer organischen Base folgen läßt.

**17.** Verfahren gemäß jedem Anspruch 1 bis 16,
dadurch **gekennzeichnet,** daß man menschliche Haare färbt.

**18.** Zusammensetzung zur Verwendung zur Färbung keratinischer Fasern,
dadurch **gekennzeichnet,** daß sie, in einem zur Färbung geeigneten Milieu, mindestens ein Salz einer seltenen Erde und mindestens ein Indolderivat der Formel (I) enthält:

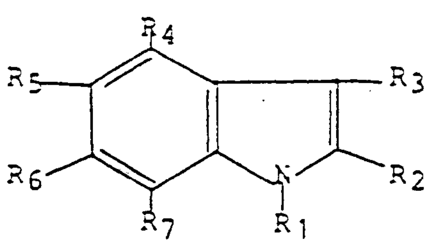

(I)

worin:

$R_1$ ein Wasserstoffatom oder eine $C_{1-4}$-Alkylgruppe darstellt;

$R_2$ und $R_3$, gleich oder verschieden, ein Wasserstoffatom, eine $C_{1-4}$-Alkyl-, Carboxyl- oder $C_{1-4}$-Alkoxycarbonylgruppe darstellen;

$R_4$, $R_5$, $R_6$ und $R_7$, gleich oder verschieden, ein Wasserstoffatom, einen $C_{1-4}$-Alkyl-, Carboxyl-, Carboxy-$C_{1-4}$-alkyl-, $C_{1-4}$-Alkoxycarbonyl-, $C_{1-4}$-Akoxycarbonyl-$C_{1-4}$-alkyl-, Carbamyl-, Halogen-, Mono- oder Polyhydroxy-$C_{1-4}$-alkyl-, $C_{1-4}$-Aminoalkylrest, eine Gruppe OZ darstellen, worin Z Wasserstoff, einen linearen oder verzweigten $C_{1-20}$-Alkylrest, eine Ar-$C_{1-4}$-alkyl-, Formyl-, lineare oder verzweigte $C_{2-20}$-Acyl-, lineare oder verzweigte $C_{3-20}$-Alkenoyl-, eine $-SiR_{11}R_{12}R_{13}$-, $-P(O)(OR_8)_2$-, $R_8OSO_2$-Gruppe bedeutet, wobei die Reste $R_4$ und $R_5$ oder auch $R_5$ und $R_6$ oder auch $R_5$ und $R_7$ zusammen mit den Kohlenstoffatomen, an die sie gebunden sind, einen Ring bilden können, der gegebenenfalls eine Carbonyl-, Thiocabonyl, $>P(O)(OR_5)$- oder $>CR_9R_{10}$-Gruppe enthält,

mit der Maßgabe, daß mindestens einer der Reste $R_4$ bis $R_7$ eine Gruppe OZ darstellt, oder daß

$R_4$ und $R_5$ oder auch $R_5$ und $R_6$ oder auch $R_6$ und $R_7$ einen Ring bilden, wobei $R_8$ und $R_9$ ein Wasserstoffatom oder eine $C_{1-4}$- Niedrigalkylgruppe darstellen, $R_{10}$ eine $C_{1-4}$-Alkoxygruppe oder eine Mono- oder Di-$C_{1-4}$-alkylaminogruppe darstellt, $R_{11}$, $R_{12}$, $R_{13}$, gleich oder verschieden, lineare oder verzweigte $C_{1-4}$-Alkylgruppen darstellen,

und die entsprechenden Salze von Alkali-, Erdalkalimetallen, von Ammonium und von Aminen, oder die Additionssalze mit Mineralsäuren oder organischen Säuren.

**19.** Zusammensetzung gemäß Anspruch 18,
dadurch **gekennzeichnet,** daß das Indolderivat 5,6-Dihydroxyindol ist.

**20.** Zusammensetzung gemäß Anspruch 18,
dadurch **gekennzeichnet,** daß das Indolderivat ausgewählt ist aus 4-Hydroxy-5-methoxyindol, 5-Methoxy-6-hydroxyindol, 2,3-Dimethyl-5,6-dihydroxyindol, 3-Methyl-5,6-dihydroxyindol und 6-Hydroxyindol.

**21.** Zusammensetzung gemäß der Ansprüche 18 bis 20,
dadurch **gekennzeichnet,** daß das Salz der seltenen Erde in Mengenverhältnissen von 0,1 bis 8 Gew.%, bezogen auf Gesamtgewicht der Zusammensetzung, vorliegt.

**22.** Zusammensetzung gemäß der Ansprüche 18 bis 20,
dadurch **gekennzeichnet,** daß das Indolderivat der Formel (I) in Mengenverhältnissen von 0,1 bis 5 Gew.%, vorzugsweise von 0,1 bis 3 Gew.%, bezogen auf Gesamtgewicht der Zusammensetzung, aufgebracht auf die Fasern vorliegt.

**23.** Zusammensetzung gemäß jedem Anspruch 18 bis 22,
dadurch **gekennzeichnet,** daß das zur Färbung geeignete Milieu zusammengesetzt ist aus einem wässrigen Milieu aus Wasser oder aus einer Mischung aus Wasser und einem organischen Lösungsmittel.

**24.** Zusammensetzung gemäß jedem Anspruch 18 bis 23,
dadurch **gekennzeichnet,** daß das zur Färbung geeignete Milieu zusammengesetzt ist aus einem im wesentlichen wasserfreien Lösungsmittelmilieu.

**25.** Zusammensetzung gemäß jedem Anspruch 18 bis 24,
dadurch **gekennzeichnet,** daß die Lösungsmittel ausgewählt sind aus Ethyl-, Propyl- oder Isopropylalkohol, t-Butylalkohol, Ethylenglycol, Propylenglycol, Monomethyl-, Monethyl- und Monobutylethern von Ethylenglycol, Monomethylethern von Propylenglycol und von Dipropylenglycol, Methyllactat.

**26.** Zusammensetzung gemäß jedem Anspruch 18 bis 25,
dadurch **gekennzeichnet,** daß sie in einem geschlossenen Gefäß im wesentlichen unter Ausschluß von Sauerstoff und in der Gegenwart eines nicht oxidierenden inerten Gases zubereitet ist.

**27.** Zusammensetzung gemäß jedem Anspruch 18 bis 26,
dadurch **gekennzeichnet,** daß sie in einer Aerosolvorrichtung in der Gegenwart eines Treibmittelgases und eines Schaumerzeugungsmittels zubereitet ist.

**28.** Zusammensetzung gemäß jedem Anspruch 18 bis 27,
dadurch **gekennzeichnet,** daß sie einen oder mehrere Hilfstoffe enthält, ausgewählt aus Fettamiden in Mengen von 0,05 bis 10%, oberflächenaktiven anionischen, kationischen, nicht-ionischen, amphoteren Mitteln oder deren Mischungen, vorhanden in Mengen von 0,1 bis 50%, aus Verdickungsmitteln, vorhanden in Mengen von 0,1 bis 5%, aus Parfüms, Sequestriermitteln, filmbildenden Mitteln, Dispergiermitteln, Konditioniermitteln, Konservierungsmitteln, opak-machenden Mitteln, aus Mitteln zur Aufblähung keratinischer Fasern.

**29.** Vorrichtung aus mehreren Teilen oder "Kit" zur Verwendung zur Färbung keratinischer Fasern und insbesondere menschlicher Haare,
dadurch **gekennzeichnet,** daß sie, in einem ersten Teil, einen Bestandteil (A), enthaltend in einem zur Färbung geeigneten Milieu mindestens ein Salz einer seltenen Erde, und in einem zweiten Teil einen Bestandteil (B), enthaltend in einem zur Färbung geeigneten Milieu mindestens ein Indolderivat der Formel (I), enthalten:

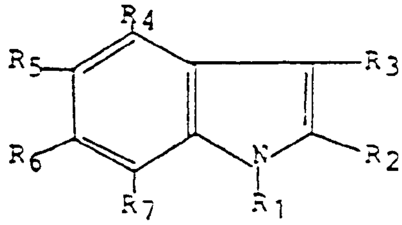

(I)

worin:

$R_1$ ein Wasserstoffatom oder eine $C_{1-4}$-Alkylgruppe darstellt;

$R_2$ und $R_3$, gleich oder verschieden, ein Wasserstoffatom, eine $C_{1-4}$-Alkyl-, Carboxyl- oder $C_{1-4}$-Alkoxycarbonylgruppe darstellen;

$R_4$, $R_5$, $R_6$ und $R_7$, gleich oder verschieden, ein Wasserstoffatom, einen $C_{1-4}$-Alkyl-, Carboxyl-, Carboxy-$C_{1-4}$-alkyl-, $C_{1-4}$-Alkoxycarbonyl-, $C_{1-4}$-Akoxycarbonyl-$C_{1-4}$-alkyl-, Carbamyl-, Halogen-, Mono- oder Polyhydroxy-$C_{1-4}$-alkyl-, $C_{1-4}$-Aminoalkylrest, eine Gruppe OZ darstellen, worin Z Wasserstoff, einen linearen oder verzweigten $C_{1-20}$-Alkylrest, eine Ar-$C_{1-4}$-Alkyl-, Formyl-, lineare oder verzweigte $C_{2-20}$-Azyl-, lineare oder verzweigte $C_{3-20}$-Alkenoyl-, eine -$SiR_{11}R_{12}R_{13}$-, -$P(O)(OR_5)_2$-, $R_5OSO_2$-Gruppe bedeutet, wobei die Reste $R_4$ und $R_5$ oder auch $R_5$ und $R_5$ oder auch $R_6$ und $R_7$ zusammen mit den Kohlenstoffatomen, an die sie gebunden sind, einen Ring bilden können, der gegebenenfalls eine Carbonyl-, Thiocabonyl, $>P(O)(OR_8)$- oder $>CR_9R_{10}$-Gruppe enthält,

mit der Maßgabe, daß mindestens einer der Reste $R_4$ bis $R_7$ eine Gruppe OZ darstellt, oder daß $R_4$ und $R_5$ oder auch $R_5$ und $R_6$ oder auch $R_6$ und $R_7$ einen Ring bilden, wobei $R_5$ und $R_9$ ein Wasserstoffatom oder eine $C_{1-4}$- Niedrigalkylgruppe darstellen, $R_{10}$ eine $C_{1-4}$-alkoxygruppe oder eine Mono- oder Di-$C_{1-4}$-Alkylaminogruppe darstellt, $R_{11}$, $R_{12}$, $R_{13}$, gleich oder verschieden, lineare oder verzweigte $C_{1-4}$-Alkylgruppen darstellen,

und die entsprechenden Salze von Alkali-, Erdalkalimetallen, von Ammonium und von Aminen, oder die Additionssalze mit Mineralsäuren oder organischen Säuren.

**30.** Vorrichtung gemäß Anspruch 29,
dadurch **gekennzeichnet,** daß sie einen dritten Teil umfaßt, der ein zur Färbung geeignetes wässriges Milieu enthält und dazu bestimmt ist, mit dem Inhalt des zweiten Teils vermischt zu werden, wenn das zur Färbung geeignete Milieu der in diesem zweiten Teil vorhandenen Zusammensetzung wasserfrei ist.

## Claims

**1.** Process for dyeing keratinous fibres, characterised in that it comprises the application:
(a) of a component (A) containing, in a medium suitable for dyeing, at least one rare-earth salt, and
(b) a component (B) containing, in a medium suitable for dyeing, at least one indole derivative of formula (I):

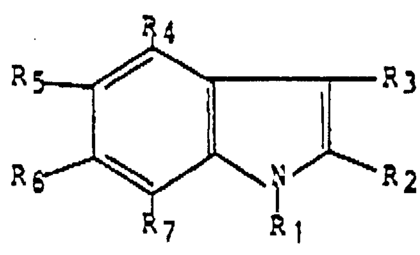

(I)

in which:

$R_1$ represents a hydrogen atom or a $C_1$-$C_4$ alkyl group;

$R_2$ and $R_3$, which are identical or different, represent a hydrogen atom, a $C_1$-$C_4$ alkyl group, a carboxyl group or a ($C_1$-$C_4$ alkoxy)carbonyl group;

$R_4$, $R_5$, $R_5$ and $R_7$, which are identical or different, represent a hydrogen atom, a $C_1$-$C_4$ alkyl, a carboxyl, a $C_1$-$C_4$ carboxylalkyl, a ($C_1$-$C_4$ alkoxy)carbonyl, a ($C_1$-$C_4$ alkoxy)carbonyl($C_1$-$C_4$ alkyl), a carbamyl,

a halogen, a $C_1$-$C_4$ mono- or polyhydroxyalkyl or a $C_1$-$C_4$ aminoalkyl radical, an OZ group in which Z denotes hydrogen, a linear or branched $C_1$-$C_{20}$ alkyl, a $C_1$-$C_4$ aralkyl group, a formyl group, a linear or branched $C_2$-$C_{20}$ acyl group, a linear or branched $C_3$-$C_{20}$ alkenoyl group, an $-SiR_{11}R_{12}R_{13}$ group, a -P(O) $(OR_8)_2$ group, an $R_8OSO_2$- group; it being possible for the radicals $R_4$ and $R_8$, or alternatively $R_5$ and $R_8$, or alternatively $R_8$ and $R_7$ to form with the carbon atoms to which they are attached, a ring optionally containing a carbonyl group, a thiocarbonyl group, a >P(O)($OR_8$) group or a >$CR_9R_{10}$ group;

provided that at least one of the radicals $R_4$ to $R_7$ represents an OZ group or alternatively that $R_4$ and $R_5$, or alternatively $R_8$ and $R_8$, or alternatively $R_6$ and $R_7$ form a ring, $R_8$ and $R_9$ represent a hydrogen atom or a $C_1$-$C_4$ lower alkyl group, $R_{10}$ represents a $C_1$-$C_4$ alkoxy group or a $C_1$-$C_4$ mono- or dialkylamino group, $R_{11}$, $R_{12}$ and $R_{13}$, which are identical or different, represent linear or branched $C_1$-$C_4$ alkyl groups,

and the corresponding salts of alkali metals, alkaline-earth metals, ammonium and amine salts, or the addition salts with inorganic or organic acids,

the components (A) and (B) being applied to the fibres by means of a single composition, or the component (A) being applied to the fibres before or after applying the component (B).

2. Process according to Claim 1, characterised in that the component (B) contains 5,6-dihydroxyindole.

3. Process according to Claim 1, characterised in that the component (B) contains a compound chosen from 4-hydroxy-5-methoxyindole, 5-methoxy-6-hydroxyindole, 2,3-dimethyl-5,6-dihydroxyindole, 3-methyl-5,6-dihydroxyindole and 6-hydroxyindole.

4. Process according to Claims 1 to 3, characterised in that in a first instance, a composition consisting of the component (A) is applied, and that after an exposure time of 1 to 30 minutes, the composition consisting of component (B), containing at least one indole derivative of formula (I) defined in Claim 1, is applied.

5. Process according to one of Claims 1 to 4, characterised in that the application of both compositions (A) and (B) is followed by treatment with hydrogen peroxide or with an inorganic or organic base.

6. Process according to any one of Claims 1 to 5, characterised in that the application of components (A) and (B) is separated by a step of rinsing the treated fibres with water.

7. Process according to any one of Claims 1 to 6, characterised in that the rare-earth salts are chosen from lanthanide salts.

8. Process according to any one of Claims 1 to 7, characterised in that the rare-earth salts are chosen from cerium $Ce^{3+}$, $Ce^{4+}$; lanthanum $La^{3+}$; europium $Eu^{2+}$, $Eu^{3+}$, gadolinium $Gd^{3+}$; ytterbium $Yb^{2+}$, $Yb^{3+}$; dysprosium $Dy^{3+}$ salts.

9. Process according to any one of Claims 1 to 8, characterised in that the rare-earth salts are chosen from rare-earth sulphates, chlorides and nitrates.

10. Process according to any one of Claims 1 to 9, characterised in that a composition (A), containing, in a medium suitable for dyeing, a cerium $Ce^{3+}$ or $Ce^{4+}$ salt, having a pH of between 2 and 12, is applied to the fibres, and in that this application is followed or preceded by the application of a solution containing at least one indole derivative of formula (I) in a medium suitable for dyeing, having a pH of between 7.5 and 12, and preferably between 8 and 11.

11. Process according to Claim 10, characterised in that the application of both compositions is followed by a treatment with a solution of hydrogen peroxide having a pH of between 2 and 12, or by a treatment with an inorganic or organic base.

12. Process according to Claim 11, characterised in that it comprises the application of a single composition containing component (A) and component (B) for an exposure time of between 1 and 30 minutes, and preferably between 1 and 15 minutes, and in that it is then rinsed with water.

13. Process according to Claim 12, characterised in that components (A) and (B) are mixed immediately before use, just before dyeing the fibres, and in that the composition is then applied in amounts effective for dyeing the keratinous fibres.

**14.** Process according to any one of Claims 1, 2, 3, 7, 8, 12 or 13, characterised in that the rare-earth salt is chosen from cerium $Ce^{3+}$ salt, europium $Eu^{2+}$ salt or ytterbium $Yb^{2+}$ salt, in sulphate or chloride form.

**15.** Process according to any one of Claims 1, 2, 3, 7, 8, 12 to 14, characterised in that a single composition, containing as component (A), a $Ce^{3+}$ sulphate or chloride and, as component (B), at least one indole derivative of formula (I), at a pH of between 2 and 7, preferably between 3 and 6, is applied to the fibres.

**16.** Process according to Claim 15, characterised in that the application is followed by a treatment with hydrogen peroxide at a pH of between 2 and 12, or by a treatment with an inorganic or organic base.

**17.** Process according to any one of Claims 1 to 16, characterised in that human hair is dyed.

**18.** Composition intended to be used for dyeing keratinous fibres, characterised in that it contains, in a medium suitable for dyeing, at least one rare-earth salt and at least one indole derivative of formula (I):

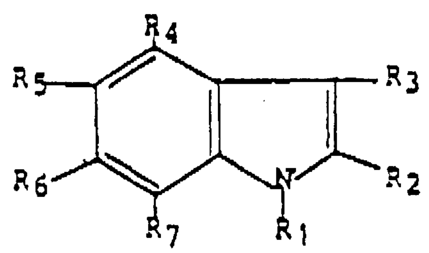

(I)

in which:

$R_1$ represents a hydrogen atom or a $C_1$-$C_4$ alkyl group;

$R_2$ and $R_3$, which are identical or different, represent a hydrogen atom, a $C_1$-$C_4$ alkyl group, a carboxyl group or a ($C_1$-$C_4$ alkoxy)carbonyl group;

$R_4$, $R_5$, $R_6$ and $R_7$, which are identical or different, represent a hydrogen atom, a $C_1$-$C_4$ alkyl, a carboxyl, a $C_1$-$C_4$ carboxyalkyl, a ($C_1$-$C_4$ alkoxy)carbonyl, a ($C_1$-$C_4$ alkoxy)carbonyl($C_1$-$C_4$ alkyl), a carbamyl, a halogen, a $C_1$-$C_4$ mono- or polyhydroxyalkyl or a $C_1$-$C_4$ aminoalkyl radical, an OZ group in which Z denotes a hydrogen, a linear or branched $C_1$-$C_{20}$ alkyl, $C_1$-$C_4$ aralkyl group, a formyl group, a linear or branched $C_2$-$C_{20}$ acyl group, a linear or branched $C_3$-$C_{20}$ alkenoyl group, an $-SiR_{11}R_{12}R_{13}$ group, a $-P(O)(OR_6)_2$ group, an $R_6OSO_2$- group; it being possible for the radicals $R_4$ and $R_6$, or alternatively $R_5$ and $R_6$, or alternatively $R_6$ and $R_7$ to form with the carbon atoms to which they are attached, a ring optionally containing a carbonyl group, a thiocarbonyl group, a $>P(O)(OR_8)$ group or a $>CR_6R_{10}$ group;

provided that at least one of the radicals $R_4$ to $R_7$ represents an OZ group or alternatively that $R_4$ and $R_5$, or alternatively $R_6$ and $R_6$, or alternatively $R_6$ and $R_7$ form a ring, $R_6$ and $R_9$ represent a hydrogen atom or a $C_1$-$C_4$ lower alkyl group, $R_{10}$ represents a $C_1$-$C_4$ alkoxy group or a $C_1$-$C_4$ mono- or dialkylamino group, $R_{11}$, $R_{12}$ and $R_{13}$, which are identical or different, represent linear or branched $C_1$-$C_4$ alkyl groups,

and the corresponding salts of alkali metals, alkaline-earth metals, ammonium and amine salts, or the addition salts with inorganic or organic acids.

**19.** Composition according to Claim 18, characterised in that the indole derivative is 5,6-dihydroxyindole.

**20.** Composition according to Claim 18, characterised in that the indole derivative is chosen from 4-hydroxy-5-methoxyindole, 5-methoxy-6-hydroxyindole, 2,3-dimethyl-5,6-dihydroxyindole, 3-methyl-5,6-dihydroxyindole and 6-hydroxyindole.

**21.** Composition according to Claims 18 to 20, characterised in that the rare-earth salt is present in proportions of between 0.1 and 8 % by weight relative to the total weight of the composition.

**22.** Composition according to Claims 18 to 20, characterised in that the indole derivative of formula (I) is present in proportions of between 0.1 and 5 % by weight, and preferably between 0.1 and 3 % by weight relative to the total weight of the composition applied to the fibres.

**23.** Composition according to any one of Claims 18 to 22, characterised in that the medium suitable for dyeing consists of an aqueous medium composed of water or a water-organic solvent mixture.

24. Composition according to any one of Claims 18 to 23, characterised in that the medium suitable for dyeing consists of an essentially anhydrous solvent medium.

25. Composition according to any one of Claims 18 to 24, characterised in that the solvents are chosen from ethyl alcohol, propyl or isopropyl alcohol, tert-butyl alcohol, ethylene glycol, propylene glycol, ethylene glycol monomethyl, monoethyl and monobutyl ethers, propylene glycol and dipropylene glycol monomethyl ethers, and methyl lactate.

26. Composition according to any one of Claims 18 to 25, characterised in that it is packaged in a closed vessel, essentially free of oxygen, and in the presence of an inert, nonoxidising gas.

27. Composition according to any one of Claims 18 to 26, characterised in that it is packaged in an aerosol device in the presence of a propellent gas and a foam generating agent.

28. Composition according to any one of Claims 18 to 27, characterised in that it contains one or more adjuvants chosen from fatty amides in proportions of 0.05 to 10 %, anionic, cationic, nonionic or amphoteric surface-active agents or mixtures thereof, which are present in proportions of between 0.1 and 50 %, thickening agents, which are present in proportions of between 0.1 and 5 %, perfumes, sequestering agents, film-forming agents, dispersing agents, conditioning agents, preserving agents, opacifying agents and keratinous fibre-swelling agents.

29. Multicompartment device or "kit" intended to be used for dyeing keratinous fibres, and more particularly human hair, characterised in that it comprises, in a first compartment, a component (A) containing, in a medium suitable for dyeing, at least one rare-earth salt; in a second compartment, a component (B) containing, in a medium suitable for dyeing, at least one indole derivative of formula (I):

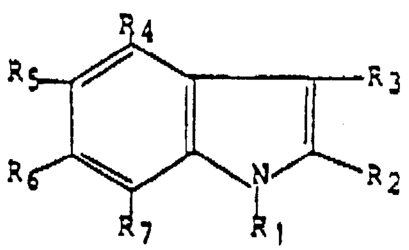

(I).

in which:

$R_1$ represents a hydrogen atom or a $C_1$-$C_4$ alkyl group;

$R_2$ and $R_3$, which are identical or different, represent a hydrogen atom, a $C_1$-$C_4$ alkyl group, a carboxyl group or a ($C_1$-$C_4$ alkoxy)carbonyl group;

$R_4$, $R_5$, $R_6$ and $R_7$, which are identical or different, represent a hydrogen atom, a $C_1$-$C_4$ alkyl, a carboxyl, a $C_1$-$C_4$ carboxyalkyl, a ($C_1$-$C_4$ alkoxy)carbonyl, a ($C_1$-$C_4$ alkoxy)carbonyl($C_1$-$C_4$ alkyl), a carbamyl, a halogen, a $C_1$-$C_4$ mono- or polyhydroxyalkyl or a $C_1$-$C_4$ aminoalkyl radical, an OZ group in which Z denotes a hydrogen, a linear or branched $C_1$-$C_{20}$ alkyl, a $C_1$-$C_4$ aralkyl group, a formyl group, a linear or branched $C_2$-$C_{20}$ acyl group, a linear or branched $C_3$-$C_{20}$ alkenoyl group, an $-SiR_{11}R_{12}R_{13}$ group, a $-P(O)(OR_6)_2$ group, an $R_8OSO_2$- group; it being possible for the radicals $R_4$ and $R_5$, or alternatively $R_6$ and $R_6$, or alternatively $R_6$ and $R_7$ to form with the carbon atoms to which they are attached, a ring optionally containing a carbonyl group, a thiocarbonyl group, a $>P(O)(OR_8)$ group or a $>CR_9R_{10}$ group;

provided that at least one of the radicals $R_4$ to $R_7$ represents an OZ group or alternatively that $R_4$ and $R_5$, or alternatively $R_6$ and $R_6$, or alternatively $R_6$ and $R_7$ form a ring, $R_6$ and $R_9$ represent a hydrogen atom or a $C_1$-$C_4$ lower alkyl group, $R_{10}$ represents a $C_1$-$C_4$ alkoxy group or a $C_1$-$C_4$ mono- or dialkylamino group, $R_{11}$, $R_{12}$ and $R_{13}$, which are identical or different, represent linear or branched $C_1$-$C_4$ alkyl groups,

and the corresponding salts of alkali metals, alkaline-earth metals, ammonium and amine salts, or the addition salts with inorganic or organic acids.

30. Device according to Claim 29, characterised in that it comprises a third compartment containing an aqueous medium suitable for dyeing and intended to be mixed with the contents of the second compartment when the medium, suitable for dyeing, for the composition present in this second compartment is anhydrous.